# EUROPEAN PATENT SPECIFICATION

(11) **EP 2 218 436 B1**
(45) Date of publication and mention of the grant of the patent: **29.05.2013**
(21) Application number: 10152932.9
(22) Date of filing: 08.02.2010
(51) Int. Cl.: A61K 8/02, A61Q 1/02, A61Q 1/08, A61Q 1/10, A61Q 1/12, A45D 33/18, A61Q 1/00, A45D 33/00

(54) **Process for the surface decoration of a cosmetic product**
Verfahren zur oberflächendekoration eines Kosmetischen Produktes
Procédé pour décorer la surface d'un produit cosmétique

(30) Priority: 17.02.2009 IT MI20090206
(43) Date of publication of application: 18.08.2010
(73) Proprietor: CHROMAVIS S.p.A., 20122 Milan (IT)
(72) Inventor: Priore, Romualdo, 20129, Milano (IT); Baroni, Alessandro, 26010, Vaiano Cremasco (CR) (IT)
(74) Representative: Vanosi, Adelio Valeriano

(56) References cited:
- US-A1- 2009 041 698
- DATABASE WPI Week 199226 Thomson Scientific, London, GB; AN 1992-209902 XP002549414 -& ES 2 027 585 A6 (AVON COSMETICS SA) 1 June 1992 (1992-06-01)
- AHN B H: "Production of multi-color powder cosmetic" WPI / THOMSON,, vol. 2003, no. 47, 26 March 2003 (2003-03-26), XP002549415 & KR 2003 0024001 A (AHN BYUNG HWA [KR]) 26 March 2003 (2003-03-26)
- DATABASE WPI Week 200401 Thomson Scientific, London, GB; AN 2004-003143 XP002549416 -& JP 2003 306412 A (TOKIWA KK) 28 October 2003 (2003-10-28)
- DATABASE WPI Week 198416 Thomson Scientific, London, GB; AN 1984-098285 XP002549417 -& JP 59 044205 A (SHISEIDO CO LTD) 12 March 1984 (1984-03-12)

## Description

The present invention relates to a method for the preparation of a decorated make-up product, in particular a baked or powdered make-up product such as eye shadow, blusher, bronzing powder or face powder.

Known cosmetic products, in particular baked products, are generally prepared by depositing a cosmetic paste of creamy (wet) form onto a base, usually of perfectly flat circular shape. The cosmetic paste is then pressed onto the base by a die.

The base with the cosmetic paste thereon is placed in an oven at atmospheric pressure, by which the contained solvent part (water) is evaporated.

Water evaporation is facilitated by the constituent material of the base, which is permeable and hence enables solvent vapours to pass during drying.

The aforedescribed baked products present a surface which is difficult to decorate by known methods of the art.

Make-up products formed from compacted powder have to be surface decorated with detailed regular patterns in order to make them aesthetically attractive and hence increase their perceived quality level (greater saleability).

Surface decorations are currently applied using various techniques. Some are based on removing material by laser or milling, from a surface previously covered by a light overspray layer. Others use double pressing (dry moulding).

"Dry moulding" is a technique able to form regular detailed patterns which penetrate into the product surface only for a maximum of 20% (extruded or compacted powders).

Overspray and laser decorations have the drawback of being removed on initial use, whereas dry moulding decorations are slightly more persistent but still remain visible only for a few applications.

The appearance of both baked and compacted powder products does not satisfy the requirements of the cosmetics market, which is always more attentive not only to the quality of the cosmetic product but also to its external appearance.

Document KR20030024001 discloses a process for manufacturing a multi-color powder cosmetic comprising the following steps:
positioning over a support a forming mold having at least one perforation;
introducing a cosmetic powder of a first colour through the perforations of the forming mold;
lowering on the forming mold a first compression mold having protrusions in correspondence with the perforations of the forming mould in order to compress the cosmetic powder of the first color;
repeating the previous steps with cosmetic powders of a second and a third color in order to obtain a multi-color compacted cosmetic powder.

An object of the present invention is to provide a method for preparing a cosmetic product which overcomes the technical drawbacks of the known art by enabling a baked cosmetic product to be decorated with other cosmetic products of different characteristics, for example chromatic.

A further object of the present invention is to provide a method for preparing a decorated cosmetic product the decoration of which does not disappear after only a few applications.

Another object is to provide a method for preparing a product with an attractive appearance which can be decorated as required, even with multi-colour decorations.

These and other objects are attained by a method for preparing a surface decorated make-up product characterized by comprising the following steps:
- depositing a predetermined quantity of a base cosmetic (3) onto a base (1A)
- positioning on the surface of the base cosmetic a first masking element (8) presenting at least one through groove (8A, 8B, 8C) formed such as to represent a required decoration
- lowering onto the first masking element a second die (9) with at least one relief (9A, 9B, 9C) provided in a position corresponding with said groove, the relief having a height such that when the die is lowered onto the first masking element it occupies at least part of the height of the underlying base cosmetic, to create therein at least one cavity corresponding with said groove; - removing the second die (9) from the first masking element (8)
- filling said cavity (10A, B, C) with a first cosmetic product (4A), and
- again lowering the second die (9) onto the first masking element such that said relief compacts the first cosmetic (4A) into said cavity
- removing the second die (9), and
- removing the first masking element (8).

Preferabily:
- said first masking element (8). presents two through grooves positioned such as to represent a required decoration
- the second die (9) presents at least one relief provided in a position corresponding with each of said grooves, such as to create in the base cosmetic at least one first and one second cavity corresponding with said first and second groove
- the first and second cavity are filled selectively with said first (4A) and a second cosmetic product
- when the second die (9) is lowered, it also compacts the first (4A) and second (4B) cosmetic product present in the first and second cavity (10A, 10B).

More in particular the step of selectively filling the first and second cavity takes place by:
- disposing on the first masking element (8) a second masking element (11) presenting at least one first through window (11A) provided to correspond to the first (10A) of said at least two grooves of the first masking element
- causing said first cosmetic (4A) product to penetrate through said first window, to hence cause it to enter said first cavity (10A).
- removing the second masking element (11) and disposing on the first masking element (12) a third masking element presenting at least one second through window (12A) provided to correspond with the second (10B) of said at least two grooves of the first masking element;
- causing said second cosmetic product to penetrate through said second window, to hence cause it to enter said second cavity, and
- removing said third masking (11) element.

In an embodiement:
- the surface of the make-up product is decorated with further cosmetic products (4C),
- said first masking element (8) comprises further grooves (10C) for each of said further cosmetic products,
- said second die (9) comprises further reliefs (9C) adapted to form further cavities within said cosmetic paste,
- said further cosmetic (4C) products are made to penetrate selectively into said further cavities, and
- are compacted therein by the reliefs (9C) of the second die.

Specifically the further cavities are selectively filled with further cosmetic products via further masking elements, each presenting further windows to correspond with said further through grooves of the first masking element.

It can be also provided that after the base (1, 2) has received the base cosmetic, this is pressed by a first die 5 before the first masking element is disposed on it.

According to an embodiment the make-up product (3, 4A, 4B, 4C) is dried after removing the first masking element

Advantageously the drying takes place in an atmospheric pressure oven at a temperature between 30ºC and 80ºC, preferably 50ºC, for a time between 8 and 24 hours, preferably 12 hours.

Optionally the drying is carried out such as to reduce the volatile component to a value less than 0.8% by weight.

According to an embodiment before drying the make-up product and after removing the first masking element, a liquid is distributed onto the product such as to amalgamate the cosmetic products and said base cosmetic.

According to a preferred embodiment before drying the make-up product and after removing the first masking element, a third die 15 is lowered onto the product such as to compact its surface.

Advantageously a porous cloth (6), preferably of microfibre, is positioned on the base cosmetic before pressing it with the first or third die.

Furthermore the first die (5) and/or the third die (15) present a flat, convex or concave surface.

According to a preferred embodiment the base cosmetic (3) deposited on the base is a wet cosmetic paste, said first and/or second cosmetic product and/or further cosmetic product being a cosmetic powder.

In another embodiment the base cosmetic deposited on the base is a cosmetic powder, said first and/or second cosmetic product and/or further cosmetic product being a wet cosmetic paste and/or a cosmetic powder.

Preferably said wet cosmetic paste presents a volatile component of between 20% and 60%, preferably 40%.

Preferably the reliefs of the second die have a height such that when it is lowered onto the masking element the reliefs do not make contact with the base, but instead a layer of base cosmetic product is present between them and the base.

In an embodiment prior to the pressing operations, at least one wall is positioned against said base (1) in order to contain said cosmetic paste during the various process steps preceding drying.

Advantageously the decorations obtained by the present invention are persistent and do not disappear after only a few applications, but remain visible until the cosmetic is almost completely used.

Further characteristics and advantages of the invention will be more apparent on reading the following description, provided by way of non-limiting example, in which:

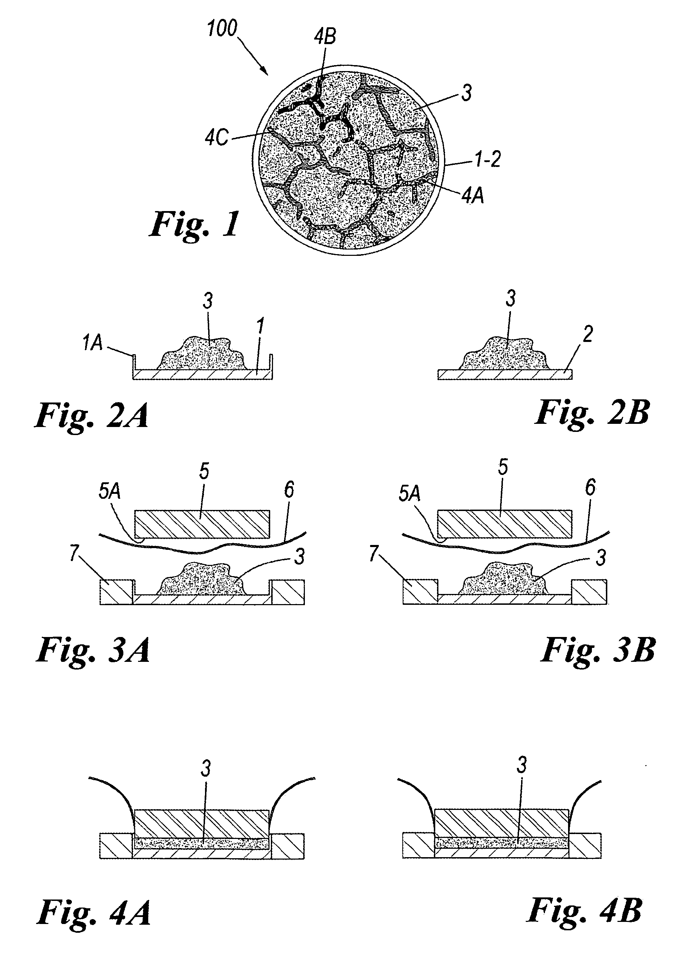

Figure 1 is a schematic view of the make-up product prepared by the method of the present invention showing on a baked paste cosmetic three decorations (4A, 4B and 4C) formed using cosmetic powders which differ from each other, for example by their colour;
Figures from 2a to 15a and from 2b to 15b show different process steps in obtaining the make-up product of Figure 1; these figures are shown in section, each process step being shown both on a traditional base (figures indicated by the reference "a") and on a flat base preferably of terracotta (figures indicated by the reference "b");
Figures 16a and 16b show in section two cosmetic products formed with a traditional and flat base respectively; and
Figure 17 shows the cosmetic product of Figure 16b housed in a closable container, which is partially shown.

The accompanying figures show a cosmetic product indicated overall by the reference numeral (100).

Figure 2a shows a traditional disc 1 having a flat bottom surface and a perimetral rim (1A), arranged to contain a base cosmetic, in this case a creamy cosmetic paste (3), which is deposited on it while still wet. The cosmetic paste presents a percentage of solvent or volatile component (in this case water) between 20% and 60%, preferably 40%.

The cosmetic paste (3) is the result of mixing water (or other volatile solvent), powders, gums, gelling agents, pearlescent powders and preservatives.

Figures 3a and 4a show a processing step in which the cosmetic paste (3) is pressed onto the base (1) by a first die (5). Advantageously the base is maintained in position by seat provided in a container (7), a cloth (6) preferably of microporous fibres being interposed between the die and the cosmetic paste (3). The die has a flat surface (5A).

The purpose of the pressing by the first die (5) (Figure 4) is to is to provide a flat surface by preforming the paste.

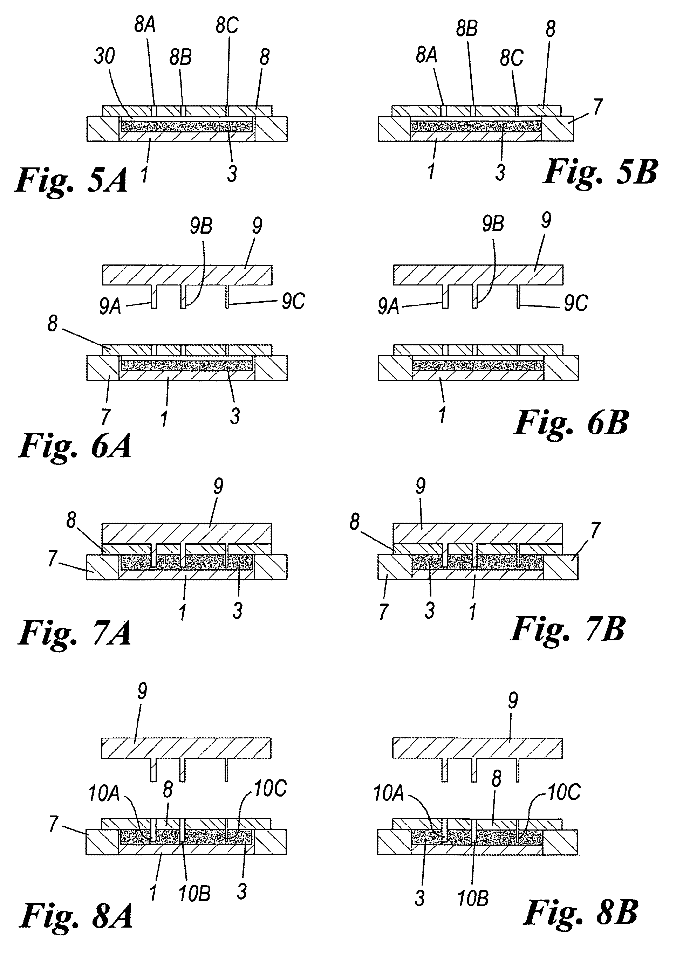

Figure 5a shows a step in which a first masking element (8) presenting a plurality of through grooves (8A, 8B, 8C) is positioned on the base (1), the grooves being positioned such as to represent a required decoration, in the case under examination this being broken branched lines (Figure 1).

Advantageously the level of the paste (3) provided in the base is such as not to completely fill it. In this respect, a free part (30) is visible between the base and masking element.

Figures 6a, 7a, 8a show a step in which a second die (9) with reliefs (9A, 9B, 9C) provided to correspond with said grooves (8A, 8B, 8C) is lowered onto the masking element (8). As can be easily seen, the reliefs have a height such that when the die 9 is lowered onto the masking element (8) (Figure 7a) they occupy at least part of the height of the underlying cosmetic paste, and advantageously are nearly in contact with the base. The pressure of the die (9) on the paste causes it to undergo complete distribution within the base so that its level rises (the space (30) disappears), to form three cavities (10A, ,10B, 10C) within the paste, each corresponding with the grooves of the masking element 8.

The cavities (10A, 10B, 10C) formed in this manner are then selectively filled each with a cosmetic product with different characteristics, for example their colour. Several cavities could however be filled with the same cosmetic product.

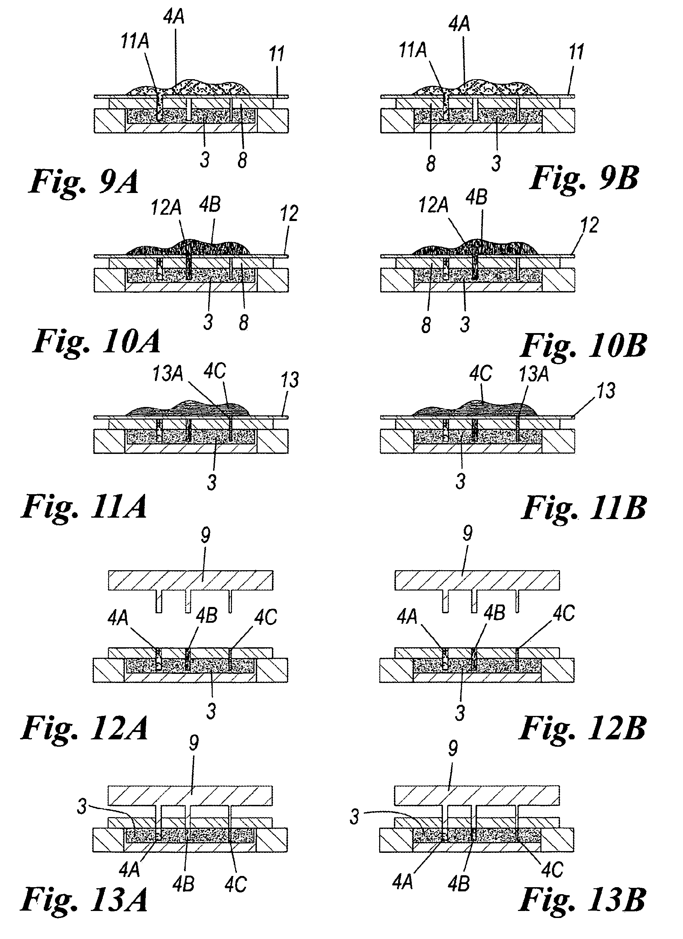

A method of selectively filling said cavities is shown in Figures 9a-11 a.

In this method, on the first masking element 8 a series of thin masking elements (11, 12, 13) are disposed in succession, these each presenting windows (11A, 12A, 13A) in positions corresponding with only one of the grooves of the masking element (8). Essentially each of the masking elements (11-13) connects the upper surface of the thin masking element, via said window, to a single groove (8A) of the masking element (8), and consequently to only one (10A) of the filled cavities within the cosmetic paste. The said masking element hence prevents access to the remaining cavities.

A first powdered cosmetic product (4A) is then deposited on the surface of the thin masking element (11) and is suitably brushed over the masking element to penetrate through the window (11A) of the thin masking element (11) and into the groove (8A) of the first masking element (8), then consequently into the first cavity (10A) of the cosmetic paste (3).

The thin masking element is then raised from the masking element (8) and the process is repeated as shown in Figures 10 and 11, with the aid of other thin masking elements (12), (13), each comprising a window in a position corresponding with only one groove of the masking element (8).

In an alternative filling method, a single masking element can present more than one window corresponding with more than one cavity of the cosmetic paste (3). In this case a single cosmetic product will fill several cavities, to hence create the required aesthetic effect.

When all the cavities (10A, 10B, 10C) have been filled (Figure 12) with the respective powdered cosmetic product (4A, 4B, 4C), the die (9) is again lowered onto the masking element (8) (Figure 13).

Advantageously the die undergoes a travel stroke such that the reliefs reach in line with the lower side of the masking element (8), to leave a substantially flat surface of the make-up product. The stroke of the die (9) could also extend to further compact the powder, to press it below the surface of the cosmetic (3).

When the die (9) and masking element (8) have been removed, the surface of the cosmetic product is impregnated with water (14) or other solvent which amalgamates the entire system.

The cosmetic paste with its inserts is then pressed by a die (15) having a suitable surface shape. Advantageously prior to pressing by the die (15) a cloth (6) preferably of porous microfibre is interposed to facilitate die detachment from the cosmetic product.

The product is then dried, for example in an atmospheric pressure oven at a temperature between 30°C and 80°C, preferably 50°C, for a time between 8 and 24 hours, preferably 12 hours.

In all cases the drying step must continue until the volatile component falls below 0.8% by weight.

On termination of the drying step the surface of the cosmetic product is cleaned by traditional methods.

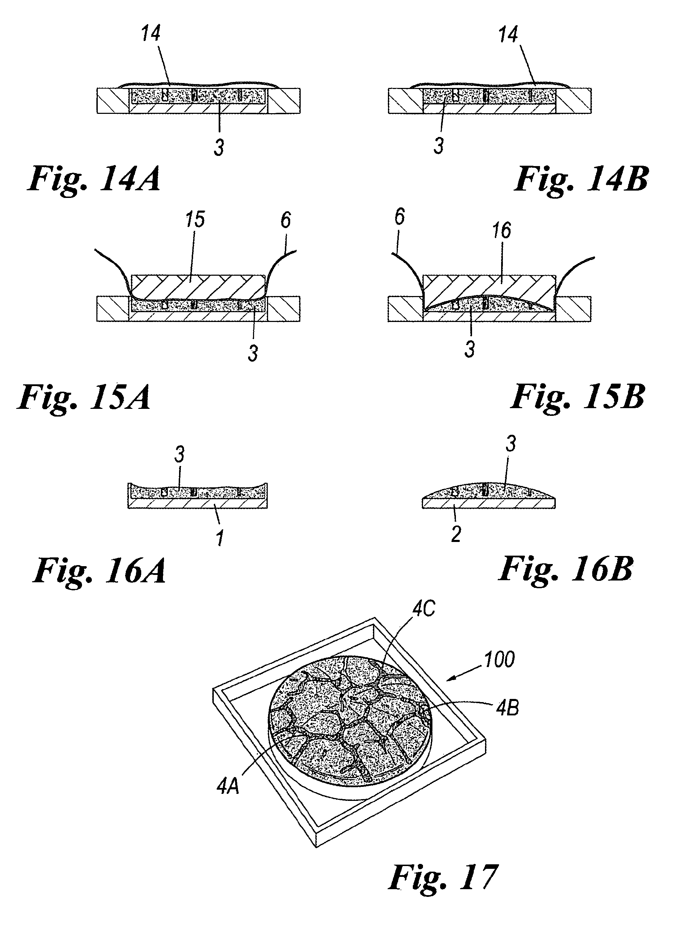

Those figures indicated by the letter b) illustrate an alternative process for obtaining a make-up product, in which the only differences from that described in the steps indicated by the letter a) consist of the fact that the base is perfectly flat and advantageously made of terracotta. Moreover in the final step (Figure 15b) the die (16) presents a concave surface. The figures in question are therefore not described, as reference can be made to those already described for understanding the processes indicated by b) in the figures.

In an alternative embodiment, the aforedescribed procedure is applied to a make-up product in which the base cosmetic (1) is not a cosmetic paste (to be then baked) but a compacted powder, while the first and second cosmetic product (and any further cosmetic products) are produced in wet (creamy) paste form.

These products in wet paste form are of conventional type, of the type already described above or for example of the type described in application MI2007A002336 in the name of the same applicant, and are to be considered entirely incorporated into this description.

In this embodiment, which is not illustrated in the drawings as any differences from that already described are only minimal, the reliefs on the second die (9) have a height which varies between 5% and 40% of the height of the underlying base cosmetic product.

The extent of cosmetic paste compaction effected by the second die is in this case very slight in order not to crack the underlying compact powder. The only reason for pressing the second die is to enable the cavities formed in the base cosmetic to be totally filled.

Advantageously the masking element (8) is very thin. In this respect when the reliefs arrive in line with the surface of the base cosmetic product the cosmetic paste present in the cavities is compacted only slightly.

In a further alternative embodiment, the aforedescribed process is applied to a make-up product in which the base cosmetic (1) is a compacted powder, and the first and second cosmetic product (and any other cosmetic products) are also in powder form.

In this case the baking step can evidently be avoided as the make-up product is immediately ready for use.

It should be noted that in the aforegoing description the term "volatile component" is used to indicate a solvent such as water, volatile silicones or the like used to amalgamate the creamy cosmetic paste.

## Claims

1. A method for preparing a surface decorated make-up product, **characterised by** comprising the following steps:
- depositing a predetermined quantity of a base cosmetic (3) onto a base (1A)
- positioning on the surface of the base cosmetic a first masking element (8) presenting at least one through groove (8A, 8B, 8C) formed such as to represent a required decoration
- lowering onto the first masking element a second die (9) with at least one relief (9A, 9B, 9C) provided in a position corresponding with said groove, the relief having a height such that when the die is lowered onto the first masking element it occupies at least part of the height of the underlying base cosmetic, to create therein at least one cavity corresponding with said groove, removing the second die (9) from the first masking element,
- filling said cavity (10A, B, C) with a first cosmetic product (4A), and
- again lowering the second die (9) onto the first masking element such that said relief compacts the first cosmetic (4A) into said cavity
- removing the second die (9), and
- removing the first masking element (8).

2. A preparation method as claimed in the preceding claim, wherein:
- said first masking element (8) presents two through grooves positioned such as to represent a required decoration
- the second die (9) presents at least one relief provided In a position corresponding with each of said grooves, such as to create in the base cosmetic at least one first and one second cavity corresponding with said first and second groove
- the first and second cavity are filled selectively with said first (4A) and a second cosmetic product
- when the second die (9) is lowered, it also compacts the first (4A) and second (4B) cosmetic product present in the first and second cavity (10A 10B).

3. A method as claimed in one or more of the preceding claims, wherein the step of selectively filling the first and second cavity takes place by:
- disposing on the first masking element (8) a second masking element (11) presenting at least one first through window (11A) provided to correspond to the first (10A) of said at least two grooves of the first masking element
- causing said first cosmetic (4A) product to penetrate through said first window, to hence cause it to enter said first cavity (10A).
- removing the second masking element (11) and disposing on the first masking element (12) a third masking element presenting at least one second through window (12A) provided to correspond with the second (10B) of said at least two grooves of the first masking element;
- causing said second cosmetic product to penetrate through said second window, to hence cause it to enter said second cavity, and
- removing said third masking (11) element.

4. A method as claimed in one or more of the preceding claims, **characterised in that**:
- the surface of the make-up product is decorated with further cosmetic products (4C),
- said first masking element (8) comprises further grooves (10C) for each of said further cosmetic products,
- said second die (9) comprises further reliefs (9C) adapted to form further cavities within said cosmetic paste,
- said further cosmetic (4C) products are made to penetrate selectively into said further cavities, and
- are compacted therein by the reliefs (9C) of the second die.

5. A method as claimed in the preceding claim, wherein the further cavities are selectively filled with further cosmetic products via further masking elements, each presenting further windows to correspond with said further through grooves of the first masking element.

6. A method as claimed in one or more of the preceding claims, wherein after the base (1, 2) has received the base cosmetic, this is pressed by a first die (5) before the first masking element is disposed on it.

7. A method as claimed in one or more of the preceding claims, wherein the make-up product (3, 4A, 4B, 4C) is dried after removing the first masking element.

8. A method as claimed in one or more of the preceding claims, wherein the drying takes place in an atmospheric pressure oven at a temperature between 30°C and 80°C, preferably 50°C, for a time between 8 and 24 hours, preferably 12 hours.

9. A method as claimed in one or more of the preceding claims, wherein the drying is carried out such as to reduce the volatile component to a value less than 0.8% by weight.

10. A method as claimed in one or more of the preceding claims, wherein before drying the make-up product and after removing the first masking element, a liquid is distributed onto the product such as to amalgamate the cosmetic products and said base cosmetic).

11. A method as claimed in one or more of the preceding claims, wherein before drying the make-up product and after removing the first masking element, a third die (15) is lowered onto the product such as to compact its surface.

12. A method as claimed in one or more of the preceding claims, wherein a porous cloth (6), preferably of microfibre, is positioned on the base cosmetic before pressing it with the first or third die.

13. A method as claimed in one or more of the preceding claims, wherein the first die (5) and/or the third die (15) present a flat, convex or concave surface.

14. A method as claimed in one or more of the preceding claims, wherein the base cosmetic (3) deposited on the base is a wet cosmetic paste, said first and/or second cosmetic product and/or further cosmetic product being a cosmetic powder.

15. A method as claimed in one or more of the preceding claims, wherein the base cosmetic deposited on the base is a cosmetic powder, said first and/or second cosmetic product and/or further cosmetic product being a wet cosmetic paste and/or a cosmetic powder.

16. A method as claimed in one or more of the preceding claims, wherein said wet cosmetic paste presents a volatile component of between 20% and 60%, preferably 40%.

17. A method as claimed in claim 1, wherein the reliefs of the second die have a height such that when it is lowered onto the masking element the reliefs do not make contact with the base, but instead a layer of base cosmetic product is present between them and the base.

18. A method as claimed in one or more of the preceding claims wherein, prior to the pressing operations, at least one wall is positioned against said base (1) in order to contain said cosmetic paste during the various process steps preceding drying.

19. A method as claimed in one or more of the preceding claims, wherein surface cleaning is carried out after the drying step.

## Patentansprüche

1. Verfahren zum Aufbereiten einer Oberfläche, eines dekorierten Make-up-Produktes,
**dadurch gekennzeichnet,**
**dass** es die folgenden Schritte umfasst:
- Auftragen einer vorbestimmten Menge einer Grundkosmetik (3) auf eine Basis (1 A)
- Anordnen auf der Oberfläche der Grundkosmetik eines ersten Maskenelements (8), welches mindestens eine durchgehende Vertiefung (8A, 8B, 8C) aufweist, welche derart ausgebildet ist, dass es eine geforderte Dekoration darstellt
- Absenken auf das erste Maskenelement eine zweite Form (9) mit mindestens einem Relief (9A, 9B, 9C), welches in einer Position, die der Vertiefung entspricht, angeordnet ist, wobei das Relief eine Höhe aufweist, so dass, wenn die Form auf das erste Maskenelement abgesenkt wird, es zumindest einen Teil der Höhe der darunter liegenden Grundkosmetik besetzt, um darin mindestens einen Hohlraum,
welcher mit der Vertiefung korrespondiert, zu erzeugen,
- Entfernen der zweiten Form (9) von dem ersten Maskenelement,
- Füllen des Hohlraums (10 A, B, C) mit einem ersten Kosmetikprodukt (4A), und
- wiederholtes Absenken der zweiten Form (9) auf das erste Maskenelement, so dass das Relief die erste Kosmetik (4A) in dem Hohlraum komprimiert
- Entfernen der zweiten Form (9), und
- Entfernen des ersten Maskenelements (8).

2. Aufbereitungsverfahren nach dem vorhergehenden Anspruch, **dadurch gekennzeichnet, dass**:
- das erste Maskenelement (8) zwei durchgehende Vertiefungen aufweist, welche angeordnet sind, um so eine geforderte Dekoration darzustellen
- die zweite Form (9) mindestens ein Relief darstellt, welches in einer Position vorhanden ist, welche mit jeder der Vertiefungen korrespondiert, um so in der Grundkosmetik mindestens einen ersten und einen zweiten Hohlraum zu erzeugen, welcher mit der ersten und der zweiten Vertiefung korrespondiert
- der erste und zweite Hohlraum selektiv mit dem ersten (4A) und einem zweiten Kosmetikprodukt gefüllt werden
- wenn die zweite Form (9) abgesenkt wird, sie auch das erste (4A) und zweite (4B) Kosmetikprodukt, welches in dem ersten und dem zweiten Hohlraum (10A, 10B) vorhanden ist, komprimiert.

3. Verfahren nach einem oder mehreren der vorhergehenden Ansprüche, **dadurch gekennzeichnet, dass** der Schritt des selektiven Füllens des ersten und zweiten Hohlraums durchgeführt wird durch:
- Anordnen auf dem ersten Maskenelement (8) ein zweites Maskenelement (11), welches mindestens ein Durchgangsfenster (11A) aufweist, welches vorhanden ist, um mit der ersten (10 A) der mindestens zwei Vertiefungen des ersten Maskenelements zu korrespondieren
- Bewirken, dass das erste Kosmetikprodukt (4A) durch das erste Fenster hindurch tritt, um dadurch in den ersten Hohlraum (10A) einzudringen
- Entfernen des zweiten Maskenelements (11) und Anordnen auf dem ersten Maskenelement (12) ein drittes Maskenelement, welches mindestens ein zweites Durchgangsfenster (12A) aufweist, welches vorhanden ist, um mit dem zweiten (10B) der mindestens zwei Hohlräume des ersten Massenelements zu korrespondieren;
- Bewirken, dass das zweite Kosmetikprodukt durch das zweite Fenster hindurch tritt, um dadurch in den zweiten Hohlraum einzudringen, und
- Entfernen des dritten Maskenelements (11).

4. Verfahren nach einem oder mehreren der vorhergehenden Ansprüche, **dadurch gekennzeichnet, dass**:
- die Oberfläche des Make-up-Produktes mit weiteren Kosmetikprodukten (4C) dekoriert wird,
- das erste Maskenelement (8) weitere Vertiefungen (10C) für jedes der weiteren Kosmetikprodukte umfasst,
- die zweite Form (9) weitere Reliefs (9C) umfasst, welche ausgestaltet sind, um weitere Hohlräume in der Kosmetikpaste auszubilden,
- dass die weiteren Kosmetikprodukte (4C) hergestellt sind, um selektiv in die weiteren Hohlräume einzudringen, und
- sie darin durch die Reliefs (9C) der zweiten Form komprimiert werden.

5. Verfahren nach dem vorhergehenden Anspruch, **dadurch gekennzeichnet, dass** die weiteren Hohlräume selektiv mit weiteren Kosmetikprodukten über weitere Maskenelemente gefüllt werden, wobei jedes weitere Fenster aufweist, um mit den weiteren durchgehenden Vertiefungen des ersten Maskenelements zu korrespondieren.

6. Verfahren nach einem oder mehreren der vorhergehenden Ansprüche, **dadurch gekennzeichnet, dass** nachdem die Basis (1, 2) die Grundkosmetik aufgenommen hat, dies durch eine erste Form (5) gedrückt wird, bevor das erste Maskenelement darauf angeordnet wird.

7. Verfahren nach einem oder mehreren der vorhergehenden Ansprüche, **dadurch gekennzeichnet, dass** das Make-up-Produkt (3, 4A, 4B, 4C) getrocknet wird, nachdem das erste Maskenelement entfernt wurde.

8. Verfahren nach einem oder mehreren der vorhergehenden Ansprüche, **dadurch gekennzeichnet, dass** das Trocknen in einem Atmosphärendruckofen bei einer Temperatur zwischen 30 °C und 80 °C, vorzugsweise bei 50 °C, über eine Zeit zwischen 8 und 24 Stunden, vorzugsweise 12 Stunden, stattfindet.

9. Verfahren nach einem oder mehreren der vorhergehenden Ansprüche, **dadurch gekennzeichnet, dass** das Trocknen ausgeführt wird, um die volatile Komponente auf einen Wert unterhalb von 0,8 Gewichtsprozent zu reduzieren.

10. Verfahren nach einem oder mehreren der vorhergehenden Ansprüche, **dadurch gekennzeichnet, dass** vor dem Trocknen des Make-up-Produktes und nach dem Entfernen des ersten Maskenelements eine Flüssigkeit auf dem Produkt verteilt wird, um die Kosmetikprodukte und die Grundkosmetik zu amalgamieren.

11. Verfahren nach einem oder mehreren der vorhergehenden Ansprüche, **dadurch gekennzeichnet, dass** vor dem Trocknen des Make-up-Produktes und nach dem Entfernen des ersten Maskenelements eine dritte Form (15) auf das Produkt abgesenkt wird, um seine Oberfläche zu komprimieren.

12. Verfahren nach einem oder mehreren der vorhergehenden Ansprüche, **dadurch gekennzeichnet, dass** ein poröses Gewebe (6), vorzugsweise eine Microfaser, auf der Grundkosmetik angeordnet wird, bevor sie mit der ersten oder dritten Form komprimiert wird.

13. Verfahren nach einem oder mehreren der vorhergehenden Ansprüche, **dadurch gekennzeichnet, dass** die erste Form (5) und/oder die dritte Form (15) eine flache, konvexe oder konkave Fläche aufweisen.

14. Verfahren nach einem oder mehreren der vorhergehenden Ansprüche, **dadurch gekennzeichnet, dass** die Kosmetik (3), welche auf der Basis aufgetragen wird, eine nasse Kosmetikpaste ist, wobei das erste und/oder zweite Kosmetikprodukt und/oder das weitere Kosmetikprodukt ein Kosmetikpulver sind.

15. Verfahren nach einem oder mehreren der vorhergehenden Ansprüche, **dadurch gekennzeichnet, dass** die Grundkosmetik, welche auf der Basis aufgetragen wird, ein Kosmetikpulver ist, wobei das erste und/oder das zweite Kosmetikprodukt und/oder das weitere Kosmetikprodukt eine nasse Kosmetikpaste und/oder ein Kosmetikpulver sind.

16. Verfahren nach einem oder mehreren der vorhergehenden Ansprüche, **dadurch gekennzeichnet, dass** die nasse Kosmetikpaste eine volatile Komponente zwischen 20 % und 60 %, vorzugsweise 40 %, aufweist.

17. Verfahren nach Anspruch 1, **dadurch gekennzeichnet, dass** die Reliefs der zweiten Form eine Höhe aufweisen, so dass, wenn sie auf das Maskenelement abgesenkt werden, die Reliefs nicht in Kontakt mit der Basis treten, sondern stattdessen eine Schicht eines Grundkosmetikprodukts zwischen ihnen und der Basis vorhanden ist.

18. Verfahren nach einem oder mehreren der vorhergehenden Ansprüche, dass vor den Druckvorgängen mindestens eine Wand gegen die Basis (1) angeordnet wird, um die Kosmetikpaste während der verschiedenen Verfahrensschritte, welche dem Trocknen vorhergehenden, zu enthalten.

19. Verfahren nach einem oder mehreren der vorhergehenden Ansprüche, **dadurch gekennzeichnet, dass** eine Oberflächenreinigung nach dem Trocknungsschritt ausgeführt wird.

## Revendications

1. Procédé pour préparer un produit de maquillage à surface décorée, **caractérisé en ce qu'**il comprend les étapes suivantes :
- le dépôt d'une quantité prédéterminée d'un cosmétique de base (3) sur une base (1A),
- le positionnement, sur la surface du cosmétique de base, d'un premier élément de masquage (8) présentant au moins une rainure traversante (8A, 8B, 8C) formée de manière à représenter une décoration requise,
- l'abaissement, sur le premier élément de masquage, d'une deuxième matrice (9) avec au moins un relief (9A, 9B, 9C) prévu à une position correspondant à ladite rainure, le relief ayant une hauteur telle que, lorsque la matrice est abaissée sur le premier élément de masquage, il occupe au moins une partie de la hauteur du cosmétique de base sous-jacent, pour créer dans celui-ci au moins une cavité correspondant à ladite rainure,
- le retrait de la deuxième matrice (9) du premier élément de masquage,
- le remplissage de ladite cavité (10A, B, C) avec un premier produit cosmétique (4A), et
- l'abaissement de nouveau de la deuxième matrice (9) sur le premier élément de masquage de sorte que ledit relief compacte le premier cosmétique (4A) dans ladite cavité,
- le retrait de la deuxième matrice (9), et
- le retrait du premier élément de masquage (8).

2. Procédé de préparation selon la revendication précédente, dans lequel
- ledit premier élément de masquage (8) présente deux rainures traversantes positionnées de manière à représenter une décoration requise,
- la deuxième matrice (9) présente au moins un relief prévu à une position correspondant à chacune desdites rainures, de manière à créer, dans le cosmétique de base, au moins une première et une deuxième cavité correspondant aux dites première et deuxième rainures,
- les première et deuxième cavités sont remplies de manière sélective avec lesdits premier (4A) et deuxième produits cosmétiques,
- lorsque la deuxième matrice (9) est abaissée, elle compacte également les premier (4A) et deuxième (4B) produits cosmétiques présents dans les première et deuxième cavités (10A, 10B).

3. Procédé selon une ou plusieurs des revendications précédentes, dans lequel l'étape de remplissage sélectif des première et deuxième cavités a lieu :
- en disposant, sur le premier élément de masquage (8), un deuxième élément de masquage (11) comportant au moins une première fenêtre traversante (11A) prévue pour correspondre à la première (10A) desdites au moins deux rainures du premier élément de masquage,
- en amenant ledit premier produit cosmétique (4A) à pénétrer à travers ladite première fenêtre, pour l'amener ainsi à entrer dans ladite première cavité (10A).
- en retirant le deuxième élément de masquage (11) et en disposant sur le premier élément de masquage (12) un troisième élément de masquage comportant au moins une deuxième fenêtre traversante (12A) prévue pour correspondre à la deuxième (10B) desdites au moins deux rainures du premier élément de masquage,
- en amenant ledit deuxième produit cosmétique à pénétrer à travers ladite deuxième fenêtre, pour l'amener ainsi à entrer dans ladite deuxième cavité, et
- en retirant ledit troisième élément de masquage (11).

4. Procédé selon une ou plusieurs des revendications précédentes, **caractérisé en ce que** :
- la surface du produit de maquillage est décorée avec d'autres produits cosmétiques (4C),
- ledit premier élément de masquage (8) comprend d'autres rainures (10C) pour chacun desdits autres produits cosmétiques,
- ladite deuxième matrice (9) comprend d'autres reliefs (9C) conçus pour former d'autres cavités dans ladite pâte cosmétique,
- lesdits autres produits cosmétiques (4C) sont amenés à pénétrer de manière sélective dans lesdites autres cavités, et
- sont compactés dans celles-ci par les reliefs (9C) de la deuxième matrice.

5. Procédé selon la revendication précédente, dans lequel les autres cavités sont remplies de manière sélective d'autres produits cosmétiques par l'intermédiaire d'autres éléments de masquage, chacun présentant d'autres fenêtres pour correspondre aux dites autres rainures traversantes du premier élément de masquage.

6. Procédé selon une ou plusieurs des revendications précédentes, dans lequel, après que la base (1, 2) a reçu le cosmétique de base, celui-ci est pressé par une première matrice (5) avant que le premier élément de masquage soit disposé sur celui-ci.

7. Procédé selon une ou plusieurs des revendications précédentes, dans lequel le produit de maquillage (3, 4A, 4B, 4C) est séché après le retrait du premier élément de masquage.

8. Procédé selon une ou plusieurs des revendications précédentes, dans lequel le séchage a lieu dans un four à pression atmosphérique à une température entre 30 °C et 80 °C, de préférence de 50 °C, pendant un temps entre 8 et 24 heures, de préférence de 12 heures.

9. Procédé selon une ou plusieurs des revendications précédentes, dans lequel le séchage est effectué de manière à réduire le composant volatile à une valeur inférieure à 0,8 % en poids.

10. Procédé selon une ou plusieurs des revendications précédentes, dans lequel, avant le séchage du produit de maquillage et après le retrait du premier élément de masquage, un liquide est distribué sur le produit de manière à amalgamer les produits cosmétiques et ledit cosmétique de base.

11. Procédé selon une ou plusieurs des revendications précédentes, dans lequel, avant le séchage du produit de maquillage et après le retrait du premier élément de masquage, une troisième matrice (15) est abaissée sur le produit de manière à compacter sa surface.

12. Procédé selon une ou plusieurs des revendications précédentes, dans lequel un tissu poreux (6), de préférence en microfibre, est positionné sur le cosmétique de base avant de le presser avec la première ou la troisième matrice.

13. Procédé selon une ou plusieurs des revendications précédentes, dans lequel la première matrice (5) et/ou la troisième matrice (15) présentent une surface plate, convexe ou concave.

14. Procédé selon une ou plusieurs des revendications précédentes, dans lequel le cosmétique de base (3) déposé sur la base est une pâte cosmétique humide, lesdits premier et/ou deuxième produits cosmétiques et/ou autres produits cosmétiques étant une poudre cosmétique.

15. Procédé selon une ou plusieurs des revendications précédentes, dans lequel le cosmétique de base déposé sur la base est une poudre cosmétique, lesdits premier et/ou deuxième produits cosmétiques et/ou autres produits cosmétiques étant une pâte cosmétique humide et/ou une poudre cosmétique.

16. Procédé selon une ou plusieurs des revendications précédentes, dans lequel ladite pâte cosmétique humide présente un composant volatile entre 20 % et 60 %, de préférence de 40 %.

17. Procédé selon la revendication 1, dans lequel les reliefs de la deuxième matrice ont une hauteur telle que, lorsqu'elle est abaissée sur l'élément de masquage, les reliefs ne font pas contact avec la base, mais au lieu de cela une couche de produit cosmétique de base est présente entre eux et la base.

18. Procédé selon une ou plusieurs des revendications précédentes, dans lequel, avant les opérations de pression, au moins une paroi est positionnée contre ladite base (1) afin de contenir ladite pâte cosmétique pendant les diverses étapes de processus précédent le séchage.

19. Procédé selon une ou plusieurs des revendications précédentes, dans lequel un nettoyage de surface est effectué après l'étape de séchage.
